(19) **Europäisches Patentamt**
**European Patent Office**
**Office européen des brevets**

(11) **EP 4 620 380 A1**

(12) **EUROPEAN PATENT APPLICATION**

(43) Date of publication:
**24.09.2025 Bulletin 2025/39**

(21) Application number: **24164713.0**

(22) Date of filing: **20.03.2024**

(51) International Patent Classification (IPC):
**A61B 5/00** (2006.01)   **A61B 5/01** (2006.01)
**A61B 5/055** (2006.01)   **A61F 7/00** (2006.01)
**G01R 33/20** (2006.01)

(52) Cooperative Patent Classification (CPC):
**A61B 5/055; A61B 5/0046; G01R 33/288;**
**A61B 5/01**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **NAUTS, Sanne**
  **Eindhoven (NL)**
• **BULUT, Murtaza**
  **Eindhoven (NL)**
• **KLAASSEN, Remy**
  **5656AG Eindhoven (NL)**
• **SPELT, Hanne Adriana Alijda**
  **Eindhoven (NL)**

(74) Representative: **Philips Intellectual Property &**
**Standards**
**High Tech Campus 52**
**5656 AG Eindhoven (NL)**

(54) **SYSTEM FOR REDUCING THERMAL DISCOMFORT DURING A MEDICAL IMAGING PROCEDURE**

(57) The invention provides a method for improving thermal comfort of a patient during a medical imaging procedure by means of varying sensorily perceptible properties of an ambient environmental state, for example by changing a color temperature and/or brightness of an illumination output provided in a space in which the scan is taking place and which is perceptible to the patient.

FIG. 1

**Description**

FIELD OF THE INVENTION

The present invention relates to the field of medical imaging, and more particularly to the reduction of patient thermal discomfort during a medical imaging procedure.

BACKGROUND OF THE INVENTION

Some medical imaging procedures require a patient to remain static for extended periods, often in unnatural circumstances or conditions. In some cases, scanning equipment may closely encroach on a patient. Examples include tomographic imaging such as MRI, CT, or PET as well as some types of extended ultrasound examination. In addition to radiography also various therapies require remaining static for extended periods in confined environments, such as radio therapy or infusion therapies.

During such procedures, the patient's temperature may increase uncomfortably due to energy deposited to the patient's body and an inability of the patient to adjust body posture in response.

For example, during MRI examinations, the physical temperature of the patient can significantly increase due to the transmitted radiofrequency field (RF) absorbed by the tissue. The amount of heating is usually represented by the Specific Absorption Rate (SAR) and expressed in Watts per kilogram (W/kg). The amount of RF emitted is dependent on the scanner field strength and type of imaging sequence. In addition, factors such as age and heart rate affect the degree of MRI-related body temperature change. See for example the following paper: Myeong Seong Kim, Investigation of Factors Affecting Body Temperature Changes During Routine Clinical Head Magnetic Resonance Imaging, Iranian Journal of Radiology.

State of the art MRI scanners may operate at high SAR to obtain better quality images. Regulations are in place to prevent a core body temperature increase greater than 1°C, limiting the SAR to 4 W/kg. However, even with these measures, patients may experience thermal discomfort as a result of RF-absorption by tissue.

Typical countermeasures to prevent a patient from becoming uncomfortable include controlling a patient's clothing so that the patient is lightly dressed, and providing bore ventilation at a sufficiently high rate.

However, a typical scan comprises a plurality of scan sequences, and the SAR associated with each can vary. This can therefore result in the patient feeling too cold during relatively low SAR sequences and the patient feeling too hot (resulting in sweating and feeling uncomfortable) during more SAR intensive sequences. Additionally, over the course of the scan, the cumulative amount of RF absorbed by tissue will increase, so that there is an underlying upwards linear trend in RF absorption, atop of which is superposed a fluctuation from sequence to sequence. Additionally, scan sequences may often be separated by pauses. During the pauses, no new energy is deposited which leads to skin temperature decreasing. The resulting cyclic variation in skin temperature during a scan has the effect of increasing thermal discomfort or thermal dissatisfaction of the patient.

SUMMARY OF THE INVENTION

The invention is defined by the claims.

According to examples in accordance with an aspect of the invention, there is provided an environmental control system for use with a medical imaging scanner, the medical imaging scanner comprising an imaging apparatus adapted to acquire imaging data of an object located in an imaging zone.

The system comprises a sensory output system comprising one or more sensory output devices adapted to generate a set of controllable sensory outputs, the combination of sensory outputs defining an ambient environment state sensorily perceptible by a patient located in the imaging zone. The sensory output system is operable to vary the ambient environment state by controlling the sensory output devices. For example, the sensory output system is operable to vary the ambient environment state by controlling one or more variable sensory parameters of the sensory outputs. Settings of the variable sensory parameters for the one or more sensory outputs may uniquely define the ambient environmental state, i.e. the ambient environmental state may be characterized by values of the one or more variable sensory parameters. The combination of sensory outputs preferably includes at least one or more visually perceptible outputs.

The ambient environment state may be for influencing a thermoregulatory state of the patient.

The system further comprises a controller.

The controller is adapted to receive scan information indicative of one or more medical imaging scan parameters as a function of time during a medical imaging scan for a patient. The scan information may include an indicator of energy deposition (or rate of energy deposition) to the patient by the scan as a function of time.

The controller is further adapted to perform a thermoperceptual compensation operation.

The thermoperceptual compensation operation may comprise determining a direct or indirect indicator of an expected body temperature change (e.g. skin temperature change) between a first time point in the medical imaging scan and a later time point in the medical imaging scan based on the scan information; and adjusting the ambient environment state at the second time point in dependence upon the expected body temperature change.

For example, adjusting the ambient environmental state may comprise estimating a change in ambient environmental state which will at least partially counter a perception of the patient of the body temperature change. For example, this may be based on a known or pre-determined relationship or correlation between changes in one or more variable sensory parameters of the ambient environmental state and changes in perceived temperature (or vice versa). For example, the known relationship or correlation may be wherein perceived body temperature increases as a function of decreasing color temperature of a light output (i.e. warmer light). For example the adjusting the ambient environmental state may comprise decreasing a color temperature of light (e.g. at the second time point) to compensate for an expected decrease in body temperature at the second time point and increasing a color temperature of light (e.g. at the second time point) to compensate for an expected increase in body temperature at the second time point. For example, in a simple case, the sensory output devices may include an illumination system for generating a light output in the ambient environment, and the adjusting the ambient environmental state may comprise increasing a color temperature of the light output (colder light color) at the second time point responsive to an expected increase in body temperature at the second time point, and decreasing a color temperature of the light output (warmer light color) at the second time point responsive to an expected decrease in body temperature at the second time point.

In some embodiments, the controller may be adapted to retrieve a pre-defined thermoperception model defining a relationship between changes in the one or more variable sensory parameters of the sensory outputs and a resultant predicted change or impact in perceived temperature by a person exposed to the ambient environment state comprising said sensory outputs (or vice versa). The resultant predicted change or impact on the perceived temperature may be qualitative, e.g. simply an indication of whether the change will increase vs decrease the (perceived) temperature, or may be an indication of a predicted quantitative change in (perceived) temperature. The controller may be adapted to adjust the ambient environmental state at the second time point based on using the thermoperception model to estimate a change in the ambient environment state setting which will at least partially counter the perception of the body temperature change.

In some embodiments, the thermoperception model may represent the relationship implicitly. For example, in some embodiments, the thermoperception model may comprise a lookup table defining mappings between different possible values for the indicator (direct or indirect) of an expected change in body temperature and settings for the ambient environment state (for the one or more variable sensory parameters of the sensory outputs) which are predicted to result in a change in perceived temperature of the user which will at least compensate for the expected change in body temperature.

In some embodiments, the at least one or more visually perceptible outputs have a controllable color content and/or controllable brightness.

Different ambient environment states have an effect on the skin body temperature, through well-established thermoregulatory pathways. These will be explained in more detail to follow.

Embodiments of the present invention are based on the concept of using a controlled amalgam of sensory outputs to influence a user's perception of temperature (e.g. thermal perception, thermal comfort or thermal satisfaction) in a controlled way, which is related to the user's physiological state (such as skin temperature, skin conductance, sweating, vasoconstriction, vasodilation, heart rate, respiration rate).

By estimating the expected body (e.g. skin surface) temperature change of the patient as a result of energy absorption by the patient, and further estimating the thermoperceptual influence of different sensory states on the patient's temperature perception, the perceived temperature change by the patient as a result of the energy absorption can be reduced or offset. Thus, the patient's thermal comfort may be increased without a need for direct thermal interaction with the patient.

As noted above, in some embodiments, a thermoperception model is employed. This may for example be stored on a datastore or memory comprised by the system.

With regards to the thermoperception model, this may for example define estimated mappings between different ambient environment state settings and a predicted change in or impact on perceived temperature by a person exposed to the ambient environment state (or vice versa). This may, by way of example, take the form of a lookup table in some embodiments, or a mathematical function in some embodiments, or a machine learning model in some embodiments.

In an advantageously simple set of embodiments, the thermoperception model may simply define mappings between possible values of the indicator of expected body temperature change and corresponding appropriate settings for the variable parameters of the ambient environmental state to achieve at least partial compensation for the temperature change.

In some embodiments, the values of the indicator of expected body temperature change, $\Delta T_p$, may be classified into a reduced set of discrete classifications, for example, low, medium and high. Each classification may correspond to a particular range of values of the indicator of expected body temperature change. The thermoperception model may define mappings between each expected body temperature change classification and a corresponding appropriate setting for variable parameters of the ambient environmental state to achieve at least partial compensation for the temperature change. Ranges of values of $\Delta T_p$ corresponding to each classification are pre-defined.

With regards to the direct or indirect indicator of an expected body temperature change, this may be a proxy indicator. In some embodiments for example, it may be a value correlated with or proportional to a rate of energy deposition to the patient by the imaging scanner at the second time point.

The medical imaging scanner may include a bore, and wherein the imaging zone is within the bore.

The medical imaging scanner may be an MRI scanner.

In some embodiments, the scan is composed of a set of individual scan sequences, wherein each scan sequence is associated with a different set of scan parameters, and wherein the first time point is a start time of the scan, and wherein the second time point is a start time (or end time) of a particular scan sequence of the scan. Thus, here, the estimated body temperature change is a body temperature change between a beginning of the scan and a start of one of the scan sequences.

Alternatively, in some embodiments the scan is composed of a set of individual scan sequences, wherein each scan sequence is associated with a different set of scan parameters, and wherein the first time point is a time point coinciding with one scan sequence, and wherein the second time point is a time point coinciding with a later scan sequence. Thus, here, the ambient environment state is adjusted so as to compensate for changes in expected body temperature between consecutive scan sequences, rather than for example total change in temperature since a start of the scan. A person's thermal comfort is typically more related to relative temperature changes in the short term rather than absolute temperature. Thus, by controlling the ambient environment state based on change in expected body temperature between consecutive scan sequences, the resulting compensatory effect will have a greater impact on the user's thermal comfort.

In some embodiments, the controller may be adapted to perform the thermoperceptual compensation operation for each scan sequence of the scan. For example, this may involve estimating an expected body temperature change induced by each scan sequence. In some embodiments, it may involve estimating a body temperature change between a first time point being a start of the scan and a plurality of second time points, each corresponding to a start or end time of a particular scan sequence of the scan. In other embodiments it may involve estimating an expected body temperature change between a series of first and second time point pairs, each pair corresponding to a pair of temporally consecutive scan sequences of the scan.

More generally, the controller may be adapted to perform the thermoperceptual compensation operation at a plurality of second time points across the duration of the scan, for example at regular time intervals throughout the scan. For instance, it could be done continuously or quasi-continuously. The first time point could be the same throughout, and the second time point changes. Alternatively, the first and second time points could move, so that the compensation operation is being performed for estimated body temperature change over a moving time window throughout the scan.

With regards to the sensory output system, the one or more sensory output devices may include an illumination system for generating a light output in the ambient environment. The illumination system may be controllable to vary a color temperature of the light output and/or to vary an illuminance or intensity of the light output. As will be explained in more detail to follow, color temperature and brightness of light are both independently correlated with changes in perceived body temperature (e.g. surface temperature, e.g. skin temperature) of a subject exposed to the light. Thus, by generating a light display and adjusting one or both of these variables of the emitted light, the subject's perceived thermal state can be influenced.

More particularly, in some embodiments, the thermoperception model may be such that light color temperature may be decreased and/or light illuminance may be increased in order to increase perceived temperature by the patient. The thermoperception model may be such that light color temperature may be increased and/or light illuminance may be decreased in order to decrease perceived temperature by the patient.

Available research shows that decrease in color temperature (meaning warmer color tones), as measured in Kelvin and/or increase in brightness leads to an increase in perceived environmental temperature (and vice versa).

As mentioned, in some embodiments, the scan may be an MRI scan.

In this case, the scan information may include an expected Specific Absorption Rate (SAR) at one or more time points during the scan. Specific Absorption Rate (SAR) is a measure of the rate at which energy is absorbed by the human body when exposed to a radio frequency (RF) electromagnetic field. It is used in MRI as a measure of rate of energy absorption by the patient as a result of the RF radiation emitted by the RF coils. It has units of energy per unit mass (mass of the patient), e.g. watts per kilogram (W/kg) or milliwatts per gram (mW/g). This energy manifests as heat when absorbed by the body.

SAR can be used to estimate an amount of energy deposited in a patient (per unit body mass) over a time period, based on multiplying the SAR of a portion of a scan (e.g. scan sequence) by the time duration of that portion of the scan. Furthermore, the SAR itself is a direct indicator of tissue heating: higher SAR has the result of greater (instantaneous) tissue heating effects. Tissue heating leads to body temperature increases. Thus, it will be seen that SAR can be used as an indicator for expected body temperature increases, for example surface body temperature increases, e.g. skin temperature increases.

As mentioned above, in some embodiments, the scan may be composed of a set of individual scan sequences. Here, the scan information may include an expected SAR for each scan sequence of the scan.

In terms of the estimated perceived temperature change, a subject's perceived temperature at a given time may be correlated with a rate of energy absorption, i.e. correlated with the value of the SAR for a given scan sequence. As discussed above, this is because the RF energy leads to local tissue warming, i.e. local increase in body temperature, e.g. increase in surface body temperature.

To explain further: to maintain a steady core body temperature, the body performs thermoregulation, a homeostatic process to keep core body temperature within a narrow range (at which all bodily processes function optimally). One set of thermoreceptors are located centrally (e.g., in the viscera). These serve to sense core body temperature. Another set of thermoreceptors are located peripherally, in the skin. These serve to sense surface temperature, also referred to as skin temperature). If thermoreceptors (centrally and/or peripherally) sense a change in temperature, a person can exhibit a physiological response (e.g., sweating, vasoconstriction/vasodilation, increasing/decreasing the metabolic rate, shivering, piloerection, etc.) and/or a behavioral response (e.g., adjusting movement, clothing, body posture or food intake/appetite; moving to another place, etc). These responses serve to keep core body temperature stable. Skin temperature is more variable (it changes more rapidly) than core body temperature, and certain thermoregulatory responses (e.g., sweating, adjusted blood flow in the skin) closely follow skin temperature. A person's thermal perception is strongly influenced by skin temperature. MR-induced RF-exposure leads to a measurable increase in skin temperature (while effects on core body temperature are present but less pronounced). This can thereby affect a person's thermal perception, thermal comfort, and thermal satisfaction.

Therefore, for simplicity, in some embodiments, the SAR itself can simply be used as a proxy indicator for at least one component of an expected body temperature change (e.g. skin or body surface temperature change), based on the observation that rate of energy absorption correlates with body temperature increase, and consequently perceived temperature. In this case for example, a change in SAR between the first time point and second time point may be used as the indicator of at least one additive component of an expected body temperature change. For example, expected SAR of an MR sequence can be used as a proxy for the body temperature change between a time before the scan has started (where SAR is zero) and a time period for which that scan sequence is being performed. Additionally or alternatively change in SAR between two consecutive scan sequences may be used as a proxy indicator of change in expected body temperature between the two scan sequences.

In addition, body temperature change (compared to a first time point) at a given time may also be correlated with a cumulative energy absorption over the time period since the first time point for which RF energy has been applied.

Thus in some embodiments, an expected SAR for each scan sequence may be used to compute an indication of an aggregate energy absorption by the patient between the first time point and the second time point, and wherein the aggregate energy absorption is used as an indicator of at least one component of the expected body temperature change.

In some embodiments, the two approaches above may be combined so that the expected body temperature change is computed as a sum of a term proportional to the aggregate energy absorption between the first and second time point and a term proportional to the change in SAR between the first time point and the second time point. The aggregate energy absorption term may represent a trending baseline in body temperature change, while the SAR may represent a local variation. The two terms may in some embodiments be weighted differently in the sum.

In some embodiments, the sensory output devices include an illumination system for generating a light output in the ambient environment. The first time point may be a time point coinciding with one scan sequence, and wherein the second time point is a time point coinciding with a later scan sequence. The thermoperception model may be such that the adjusting the ambient environment state at the second time point in dependence upon the expected body temperature change comprises changing a color temperature of a light output by an amount which is a function of the change in SAR between the one scan sequence and the later scan sequence.

In some embodiments, the thermoperception model defines mappings between the SAR of a given scan sequence and color temperature of light to output during the scan sequence, and wherein the mappings are such that color temperature of light to be applied increases as a function of increasing SAR.

In some embodiments, the sensory output system may comprise a display device for presenting a 2D visual output, e.g. a display panel or projector.

The 2D visual output may comprise visual elements having a perceptible color temperature, and wherein the color temperature is adjustable.

In some embodiments, the 2D visual output may comprise a presentation of visual imagery having perceptible color content, and wherein adjusting the color temperature of the output comprises adjusting the visual imagery.

For example imagery may be shown that is associated with coldness (e.g., snowy landscapes, cool water running down a stream) or warmth (e.g., a tropical beach, a fireplace with crackling fire).

Additionally or alternatively, the 2D visual output may comprise a presentation of visual imagery having a conceptual association with a range of thermal temperatures. In this case, an effect is induced by virtue of the content of the scene being associated with warmth (a hot sweater, cup of hot tea, hot tub, etc.) or coldness. This can be associated with a specific color, but does not have to be.

In some embodiments, the sensory output devices may include an audio output device. Audio content can be adapted to fit a target change in perceived temperature. For example, if warm elements are displayed on a display device, audio associated with these elements can be generated (e.g., crackling fire, running cold water, opening a fresh drink, etc.).

In some embodiment, the display device may be controlled to display visual representations of transitions between contrasting temperature scenes.

Here, the intention is to evoke a shift in the patient's thermoceptive state based on the effect of contrast. For example, to guide a patient to move from one thermoperceptual state (e.g., feeling hot) to another (e.g., feeling less hot), scenes might be displayed which portray successful resolution of an aversive thermoceptive state. As an example, when a patient feels cold, a display screen might show a scene in which a person (or cartoon character) is shivering and looking cold before putting on a sweater, sitting by the fire and drinking hot chocolate. Alternatively, if a person is feeling hot, the display screen could show a scene of a person sweating and looking hot before diving into cold water on a sunny day.

In some embodiments, the sensory output devices may include an olfactory output device. The patient might be exposed to a smell associated with warmth, e.g., the smell of coconut, hot chocolate, or a campfire, or associated with coldness.

In some embodiments, the sensory output devices may include a tactile stimulus generator. For example, tactile stimuli can be used to distract a patient from their aversive thermoceptive state. Tactile sensations such as vibration or tapping can alleviate feelings of thermal discomfort.

As mentioned above, in some embodiments, the change in the ambient environment state may comprise at least a change in color temperature of a light output generated by an illumination system. In this case, in some embodiments, the controller may be adapted to control the sensory output system such that a transition from a first ambient environment state to a second ambient environment state comprises a transition of the light output through a path in color space. The transition may be a smooth transition of the light output through color space in some embodiments.

One possible example of this is as follows. The transition in the ambient environment state may comprise at least a transition in RGB color of an RGB light output of the illumination system from a first color (A) in RGB space to a second color (B) in RGB space. Performing the transition from color A to color B may comprise: converting color A and color B into points in the CIELAB color space; determining a continuous line through CIELAB color space between points A and B; extracting a series of CIELAB color points lying along the CIELAB line and converting the extracted series of points to RGB color space, to thereby derive a series of RGB color points; controlling the illumination system to transition between color A and color B by controlling the illumination system to sequence through the series of RGB color points. RGB is not a perceptually uniform color space therefore transitions from color A(R1,G1,B 1) to B(R2, G2, B2) will seem abrupt to a human observer. Thus, to smooth the transition, the transition function can be defined in CIELAB color space before being converted back into RGB color space.

In some embodiments, to improve estimations of expected body temperature change, the system may further comprise one or more temperature sensors for sensing a body temperature of the patient. The controller may be adapted to determine the indicator of an expected body temperature change based on at least a combination of the scan information and a measure of patient body temperature at the second time point.

The system may further comprise one or more ambient temperature sensors for sensing a temperature of the ambient environment. The controller may be adapted to determine the indicator of an expected body temperature change based on at least a combination of the scan information and a measure of ambient environment temperature at the second time point.

A combination of body and ambient environment temperature sensors may be used in some embodiments.

In some embodiments, the system may further comprise one or more physiological parameter sensors for sensing one or more physiological parameters of the patient. The controller may be adapted to determine the indicator of an expected body temperature change based on at least a combination of the scan information and measurements of the one or more physiological parameters at the second time point.

These may include for example heart rate or sweat rate.

In some embodiments, the determining the estimated body temperature change may further be based on one or more biological parameters of the patient, such as any one or more of: age, gender, body mass, height, weight, body composition, metabolic base rate, resting heart rate, average activity level, sleep patterns.

In some embodiments, the controller may be adapted to determine the indicator of expected body temperature change based on use of a pre-defined thermophysiological model.

For example, where the scan is an MRI scan, and the scan information includes an expected Specific Absorption Rate (SAR) at one or more time points during the scan, the controller may be adapted to determine the indicator of expected body temperature change based on use of a pre-defined thermophysiological model adapted to estimate RF-induced body temperature increases as a function of SAR and exposure duration.

Additional factors, such as ambient temperature, clothing, patient size and position, coil size and position, patient ventilation information, and/or the individual's thermoregulatory capabilities may also be incorporated as variables in the model.

In some embodiments, the controller may be further adapted to receive patient-specific temperature sensitivity information and wherein the determining the indicator of expected body temperature change is performed in part based on the patient-specific temperature sensitivity information.

In some embodiments, the controller is further adapted to receive user input at the second time point indicative of patient-reported temperature perception and to adjust the ambient environment state based in part on the user input.

Another aspect of the invention is an environmental control method for use during a medical imaging scan with a medical imaging scanner, where the medical imaging scanner comprised an imaging apparatus adapted to acquire imaging data of an object located in an imaging zone.

The method may involve control of a sensory output system, the sensory output system comprising one or more sensory output devices adapted to generate a set of controllable sensory outputs, the combination of sensory outputs defining an ambient environment state sensorily perceptible by a patient located in the imaging zone. The sensory output system is operable to vary the ambient environment state by controlling the sensory output devices. For example, the sensory output system is operable to vary the ambient environment state by controlling one or more variable sensory parameters of the sensory outputs. Settings of the variable sensory parameters for the one or more sensory outputs may uniquely define the ambient environmental state, i.e. the ambient environmental state may be characterized by values of the one or more variable sensory parameters. The combination of sensory outputs preferably includes at least one or more visually perceptible outputs.

The ambient environment state may be for influencing a thermoregulatory state of the patient.

The method may comprise the following steps: receiving scan information indicative of medical imaging scan parameters as a function of time during a medical imaging scan for a patient. The scan information may include an indicator of energy deposition (or rate of energy deposition) to the patient by the scan as a function of time.

The method may further comprise performing a thermoperceptual compensation operation.

The thermoperceptual compensation operation may comprise: determining a direct or indirect indicator of an expected body temperature change between a first time point in the medical imaging scan and a second later time point in the medical imaging scan based on the scan information, and adjusting the ambient environment state at the second time point in dependence upon the expected body temperature change.

For example, the adjusting the ambient environmental state may comprise estimating a change in ambient environmental state which will at least partially counter a perception of the temperature change by the patient. For example, this may be based on a known or pre-determined relationship or correlation between changes in one or more variable sensory parameters of the ambient environmental state and changes in perceived temperature.

In some embodiments, the method may comprise retrieving a pre-defined thermoperception model defining a relationship between changes in the one or more variable sensory parameters of the sensory outputs and a resultant predicted change or impact in perceived temperature by a person exposed to the ambient environment state comprising said sensory outputs. The predicted change or impact may be qualitative, e.g. simply an indication of whether the change will increase vs decrease the perceived temperature, or may be an indication of a predicted quantitative change in perceived temperature. The controller may be adapted to adjust the ambient environmental state at the second time point based on using the thermoperception model to estimate a change in the ambient environment state setting which will at least partially counter the patient's perception of the expected body temperature change.

Any feature or embodiment described in relation to the first (apparatus) aspect of this invention may also be applied or incorporated as an embodiment of the method aspect of this invention.

Another aspect of the invention is a computer program product comprising computer program code configured, when executed by a processor, to cause the processor to perform a method in accordance with any example or embodiments described in this document or in accordance with any claim of this application. For example, the processor may be coupled to a datastore which stores a representation of the thermoperception model.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example controller and system in accordance with one or more embodiments of the invention;

Fig. 3 illustrates an example of adjustment of an ambient environmental state for each of a series of scan sequences of a medical imaging scan; and

Fig. 4 illustrates an example of adjustment of an ambient environmental state for each of a series of scan sequences of a medical imaging scan, the scan sequences separated by time delays.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for improving thermal comfort of a patient during a medical imaging procedure by means of varying sensorily perceptible properties of an ambient environmental state, for example by changing a color and/or brightness of an illumination output provided in a space in which the scan is taking place and which is perceptible to the patient.

One advantageous application is for use during MRI examinations, as will be explained below. However, the same principle can be applied for any type of medical imaging examination.

By way of background, during MRI examinations, the body temperature of the patient can significantly increase, due to the transmitted radiofrequency field (RF) being absorbed by the patient's tissue. The amount of heating can be quantitatively represented by the Specific Absorption Rate (SAR). Specific Absorption Rate (SAR) is a measure of the rate at which energy is absorbed by the human body when exposed to a radio frequency (RF) electromagnetic field. It is used in MRI as a measure of rate of energy absorption by the patient as a result of the RF radiation emitted by the RF coils. It has units of energy per unit mass (mass of the patient), e.g. watts per kilogram (W/kg) or milliwatts per gram (mW/g).

The amount of RF energy emitted is dependent upon the scanner field strength and type of imaging sequence. In addition, patient-dependent factors such as age and body size also impact MR-related body temperature change. Reference is made in this regard to the following paper: Myeong Seong Kim, Investigation of Factors Affecting Body Temperature Changes During Routine Clinical Head Magnetic Resonance Imaging, Iranian Journal of Radiology.

State of the art MRI scanners operate with a high SAR to achieve higher quality images. Regulations are in place to prevent core body temperature increase greater than 1 °C, limiting the SAR to a maximum of 4 W/kg in certain regions. However, even with these measures, patients may experience thermal discomfort as a result of RF-absorption by tissue.

MR-induced RF exposure mainly leads to an increase in temperature at the surface of the body. For example, reference is made to the following paper: ADAIR, E.R. and BERGLUND, L.G. (1992), Predicted Thermophysiological Responses of Humans to MRI Fields. Annals of the New York Academy of Sciences, 649: 188-200. This showed that, for example, after three successive 20-minute MRI-exams with high SAR, an increase in skin temperature was observable of 3.5 °C, along with a small increase in core body temperature of 0.3 °C. In this study, increase in skin temperature was followed by increase in perceived temperature, sweating & skin blood flow.

Reference is further made to the paper: Schlader et al, "Human temperature regulation when given the opportunity to behave", Eur J Appl Physiol (2013) 113:1291-1301. This study found that changes in skin temperature have a strong impact on thermal comfort.

It has previously been found that the sensorily perceptible characteristics of an ambient environment, sometimes referred to as Ambient Experience or Ambient Environment, can influence perceived temperature changes.

For example, reference is made to the following paper: Hoffman, H. G., Richards, T. L., Coda, B., Bills, A. R., Blough, D., Richards, A. L., & Sharar, S. R. (2004). Modulation of thermal pain-related brain activity with virtual reality: evidence from fMRI. Neuroreport, 15(8), 1245-1248. This study found that thermal discomfort can be partially compensated by adjusting lighting or visuals, e.g., by playing a VR game displaying a snowy landscape (Snow World).

Reference is further made to the following paper: Mogensen, M. F., & English, H. B. (1926). The apparent warmth of colors. The American Journal of Psychology. This details the so-called hue-heat effect, which suggests that perceived temperature is influenced by color.

For example, it has been found in a further study that people feel warmer in a space illuminated with warm ambient colors, i.e. lower color temperature (e.g. color temperature 3000K), and/or at high illumination (e.g. 800 lx), compared to space illuminated with cool ambient colors, i.e. higher color temperature (e.g. color temperature 5500 K) at lower illumination levels (e.g. 300 lx), even when the objective temperature of both rooms is identical. Thus study is detailed in the following paper: Tsushima, Y., Okada, S., Kawai, Y., Sumita, A., Ando, H., & Miki, M. (2020). Effect of illumination on perceived temperature. Plos one, 15(8), e0236321.

A review summarizing 31 experiments is presented in the paper: Harry S. Mayes, Martina Navarro, Liam P. Satchell, Michael J. Tipton, Soichi Ando, Joseph T. Costello, "The effects of manipulating the visual environment on thermal perception: A structured narrative review", Journal of Thermal Biology, Volume 112, 2023 (herein, "Mayes et al"). The review suggests that thermal perception and thermal comfort are affected by changes in lighting.

There are two main theories which both indicate that light color is correlated with changes in perceived temperature and thermal comfort.

According to the Hue-Heat Hypothesis people associate red (and reddish colors) with warmth, and blue (and blueish colors) with coolness; seeing 'warm' colors makes people feel more warm, while seeing 'cool' colors makes people feel more cool. The Hue-Heat Hypothesis is described further in the following paper: Kulve et al, The influence of light on thermal responses, Acta Physiol., 216 (2) (2015), pp. 163-185.

The Correlated Color Temperature theory posits the same hypothesis, but relating specifically to light; measuring color temperature in Kelvin (K). As such, light with a lower degree of Kelvin is more red, and therefore, associated with warmth. Likewise, light with higher degree of Kelvin is more blue, and therefore associated with coolness. The correlated color temperature theory is further outlined in the following paper: Golasi et al., "Influence of lighting color temperature on indoor thermal perception: a strategy to save energy from the HVAC installations", Energy Build., 185 (2019), pp. 112-122.

As detailed in (Mayes et al, 2023), the color temperature of light has been found to affect thermal perception, thermal comfort, skin temperature, and thermoregulatory responses. The color of light has been found to affect both physiological thermoregulatory responses (e.g., heart rate, skin blood flow, sweating, shivering), as well as behavioral responses (e.g., moving from one room to another; putting on more or less clothing).

Thus, the effect of light on perceived temperature is at least in part mediated through involuntary impacts of the light on the thermoregulatory system of the subject (i.e. light stimulates autonomic responses in the patient which impact skin temperature and/or core body temperature. In this regard, reference is made to section 3.7 of (Mayes et al, 2023).

From the above discussion, it can be concluded that light and visuals can have a substantial effect on perceived temperature, and that adjusting light (e.g., color, brightness) may be used to alleviate thermal discomfort. For example, increasing color temperature of light (making the light 'cooler' in color) has the effect of decreasing perceived body temperature of the subject, and decreasing color temperature of the light (making the light 'warmer') has the effect of increasing perceived body temperature of the subject.

Accordingly, it is proposed by the inventors of the present invention to use controlled adjustment of sensorily perceptible characteristics of an ambient environment (sometimes referred to as Ambient Experience (AE)) to help patients better manage temperature-related discomfort, by adapting the AE in line with expected, measured, or perceived temperature changes.

For example, considering the particular application of MRI imaging, ambient experience may be adjusted as a means of influencing how patients perceive temperature inside the bore of the scanner. Based on this, in accordance with one set of embodiments, it is proposed to adapt the ambient experience settings (e.g. light, and optionally also video and sound) based on MRI scan sequence information, and more specifically based on the expected body temperature change per specific MR sequence.

In accordance with one particular set of embodiments, it is proposed to use the expected specific absorption rate (SAR) as a proxy for perceived temperature changes by the patient undergoing the scan, and to adjust ambient light conditions in line with the expected body temperature change, to thereby compensate or negate or counter the patient's perceived temperature change (e.g., adapting the brightness and color of light depending on the SAR deposited during a specific scan sequence, and/or during the total examination).

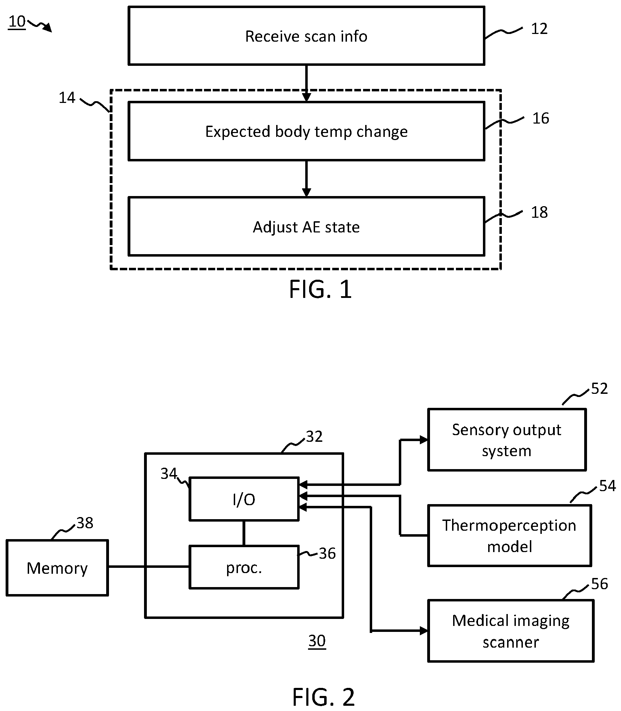

Fig. 1 outlines in block diagram form steps of an example method according to one or more embodiments. The steps will be recited in summary, before being explained further in the form of example embodiments.

The method 10 is an environmental control method for use during a medical imaging scan with a medical imaging scanner. For example, the medical imaging scanner may comprise an imaging apparatus adapted to acquire imaging data of an object located in an imaging zone.

The method 10 comprises control of a sensory output system, the sensory output system comprising one or more sensory output devices adapted to generate a set of controllable sensory outputs, and the combination of sensory outputs defining an ambient environment ("AE") state sensorily perceptible by a patient located in the imaging zone. The sensory output system is operable to vary the ambient environment state by controlling the sensory output devices. For example, the sensory output system is operable to vary the ambient environment state by controlling one or more variable sensory parameters of the sensory outputs. Settings of the variable sensory parameters for the one or more sensory outputs may uniquely define the ambient environmental state, i.e. the ambient environmental state may be characterized by values of the one or more variable sensory parameters.

The ambient environment state may be for influencing a thermoregulatory state of the patient and/or a thermoperceptual state of the patient.

The combination of sensory outputs preferably includes at least one or more visually perceptible outputs.

The method 10 comprises receiving 12 scan information indicative of medical imaging scan parameters as a function of time during a medical imaging scan for a patient. The scan information may include an indicator of energy deposition (or rate of energy deposition) in the patient associated with the scan as a function of time.

The method may optionally further comprise retrieving a thermoperception model defining a relationship between changes in the ambient environmental state and resultant predicted changes or impact in perceived temperature by a person exposed to the ambient environment state. For example, the thermoperception model may define a relationship between changes in the one or more variable sensory parameters of the sensory outputs and a resultant predicted change or impact in perceived temperature by a person exposed to the ambient environment state comprising said sensory outputs. The predicted change or impact may be qualitative, e.g. simply an indication of whether the change will increase vs decrease the perceived temperature, or may be an indication of a predicted quantitative change in perceived temperature.

In some embodiments, the thermoperception model may define estimated mappings between different ambient environment state settings and a change (e.g. a quantitative impact) in perceived temperature by a person exposed to the ambient environment state.

In some embodiments, the thermoperception model may be configured to output an estimate of a change of ambient environment state setting(s) which will result in a change in perceived temperature of the user which will compensate for a given expected change in actual body temperature, for example actual skin temperature. Such a thermoperception model could be implemented using a simple lookup table, wherein the lookup table permits mapping from a value indicative of predicted change in body temperature to a particular set of settings for the ambient environmental state for compensating for the change, for example including at least a setting for a color temperature of an illumination output.

The method may further comprise performing a thermoperceptual compensation operation 14. The thermoperceptual compensation operation 14 comprises determining 16 a direct or indirect indicator of an expected body temperature change between a first time point in the medical imaging scan and a later time point in the medical imaging scan based on the scan information.

The thermoperceptual compensation operation 14 further comprises adjusting 18 the ambient environment state at the second time point in dependence upon the expected body temperature change.

For example, the adjusting 18 the ambient environmental state may comprise estimating a change in ambient environmental state which will at least partially counter the patient's perception of the body temperature change. For example, this may be based on a known or pre-determined relationship or correlation between changes in one or more variable sensory parameters of the ambient environmental state and changes in perceived temperature.

For example, in some embodiments, the adjusting the ambient environmental state may comprise decreasing a color temperature of light (e.g. at the second time point) to compensate for an expected decrease in body temperature at the second time point and increasing a color temperature of light (e.g. at the second time point) to compensate for an expected increase in body temperature at the second time point. For example, in a simple case, the sensory output devices may include an illumination system for generating a light output in the ambient environment, and the adjusting the ambient environmental state may comprise increasing a color temperature of the light output (colder light color) at the second time point responsive to an expected increase in body temperature at the second time point, and decreasing a color temperature of the light output (warmer light color) at the second time point responsive to an expected decrease in body temperature at the second time point.

Where a thermoperception model is used, the controller may be adapted to adjust the ambient environmental state at the second time point based on using the thermoperception model to estimate a change in the ambient environment state setting which will at least partially counter the patient's perception of the body temperature change.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example controller 32 configured to execute a method in accordance with one or more embodiments of the invention. The controller is shown in the context of a system 30 which comprises the controller. The controller alone represents an aspect of the invention. The system 30 is another aspect of the invention. The provided system need not comprise all the illustrated hardware components; it may comprise only a subset of them.

The controller 32 comprises one or more processors 36 configured to perform a method in accordance with that outlined above, or in accordance with any embodiment described in this document or any claim of this application. In the illustrated example, the controller further comprises an input/output (I/O) 34 or communication interface.

In the illustrated example of Fig. 2, the system 30 comprises a sensory output system 52. The sensory output system 52 comprises one or more sensory output devices adapted to generate a set of controllable sensory outputs, the combination of sensory outputs defining an ambient environment (AE) state sensorily perceptible by a patient located in the imaging zone. The sensory output system is operable to vary the ambient environment state by controlling the sensory output devices. For example, the sensory output system is operable to vary the ambient environment state by controlling one or more variable sensory parameters of the sensory outputs. The combination of sensory outputs includes at least one or more visually perceptible outputs.

In the illustrated example of Fig. 2, the system 30 further comprises an (optional) thermoperception model 54 adapted to define a relationship between changes in the one or more variable sensory parameters of the sensory outputs and a resultant predicted change or impact in perceived temperature by a person exposed to the ambient environment state comprising said sensory outputs.

For example, in some embodiments, the thermoperception model 54 may define estimated mappings between different ambient environment state settings and a change in perceived temperature by a person exposed to the ambient environment state. The thermoperception model may be stored or embodied on a memory or datastore for example.

In the illustrated example of Fig. 2, the system further comprises a medical imaging scanner 56 comprising an imaging apparatus adapted to acquire imaging data of an object located in an imaging zone. For example, this may be an MRI scanner. However, the scanner is not an essential component of the system.

The system 30 may further comprise a memory 38 for storing computer program code (i.e. computer-executable code) which is configured for causing the one or more processors 36 of the controller 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

An example implementation of the method according to a particular set of embodiments will now be described by way of illustration of the above-summarized concept of the invention. It will be appreciated that not all features of this particular set of embodiments are essential to the inventive concept, and are described to aid understanding and to provide an example to illustrate the inventive concepts.

In accordance with this particular set of embodiments, the imaging scan may be composed of a plurality of scan portions, wherein the scan parameters associated with each portion may differ.

In particular, by way of one example, the scan may be composed of a set of individual scan sequences, each scan sequence associated with a different set of scan parameters. For example, in MRI imaging, an MRI examination (planned as an "exam card") typically consists of multiple scan sequences. Inter alia, the specific absorption rate (SAR) of these different sequences can differ from sequence to sequence. Additionally, over the course of the Exam Card, the cumulative amount of RF energy absorbed by tissue will increase. Consequently, there is an upwards linear trend in deposited RF energy, on top of a temporally local fluctuation from sequence to sequence.

As a consequence, it is the recognition of the inventors that it may be valuable to vary the ambient environment state throughout the scan such that the ambient environmental state during each scan sequence is specifically adjusted to compensate for the estimated perceived thermal burden on the patient during that scan sequence.

By way of example, with reference to the thermoperceptual compensation operation, it is proposed that the first time point may be a start time of the scan, and the second time point may be a start time or end time of a particular scan sequence of the scan. Alternatively, the first time point may be a time coinciding with one scan sequence and the second time point may be a time coinciding with a later scan sequence, for example a temporally next scan sequence.

It is preferred in accordance with this set of embodiments that the controller is adapted to perform the thermoperceptual compensation operation for each scan sequence of the scan based on estimating an expected body temperature change induced by each scan sequence, compared either to the start of the scan or the previous scan sequence. To this end, the controller may be adapted to perform the thermoperceptual compensation operation at a plurality of second time points across the duration of the scan, for example one for each scan sequence. Additionally or alternatively, the thermoperceptual compensation operation may be performed at regular time intervals throughout the scan, where the time interval may be shorter than the total duration of any individual scan sequence. In this way, temperature compensation may for example be updated continuously or quasi-continuously.

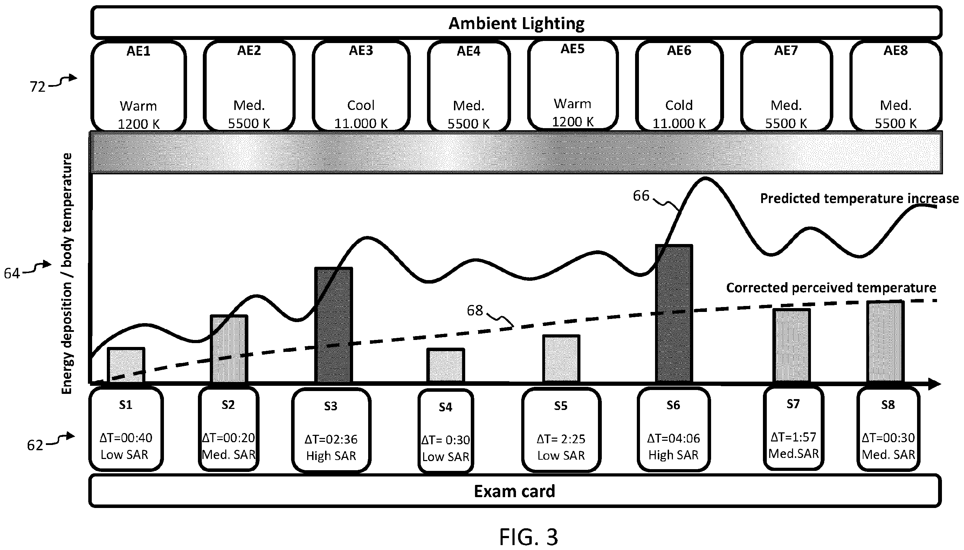

The proposed implementation in accordance with the presently described set of embodiments in illustrated schematically by Fig. 3.

Fig. 3 illustrates an exemplary MRI scan composed of an ordered series of eight scan sequences. In medical imaging such as MRI or CT, a scan is typically composed of an ordered series of different scan sequences, where each scan sequence has adjusted scan parameters for acquiring images of different contrasts or resolutions, or to highlight different types of tissues or pathologies. In the particular context of MRI, the term 'scan sequence' refers to the particular series of magnetic pulses and gradients applied to the body of the patient to acquire the targeted type of images. Examples of different scan sequences in the context of MRI include for instance: T1-weighted images, T2-weighted images, proton density images, diffusion-weighted images.

The series of scan sequences forming the scan are indicated by arrow 62 in Fig. 3 and are labelled S 1-S8. Each scan sequence is labelled in accordance with its SAR, either low SAR, medium SAR or high SAR. S1 is low SAR, S2 is medium SAR, S3 is High SAR, S4 is low SAR, S5 is low SAR, S6 is high SAR, S7 is medium SAR and S8 is medium SAR. By way of example, a high SAR might correspond to an SAR of between 2-4 W/kg, a medium SAR might correspond to an SAR of between 0.5-2 W/kg and a low SAR might correspond to an SAR of less than 0.5 W/kg. The time duration, $\Delta T$, of each sequence is further indicated for each scan sequence.

Above the outline of the scan sequences in Fig. 3 is a schematic bar graph 64 illustrating rate of energy deposition to the patient during each of the scans, i.e. the SAR. The height of each bar represents the rate of energy deposition to the patient during the particular one of the scan sequences 62 with which the bar is aligned.

Also illustrated in Fig. 3 is a line 66 indicating a predicted perceived temperature increase by the patient at each of a series of (second) time points throughout the scan relative to a first time point corresponding to a start time (i.e. t=0) of the scan. It can be seen that the perceived temperature increase trends gradually upwards throughout the scan as energy is cumulatively deposited in the patient by the RF emissions, and at the same time there is local fluctuation in the perceived temperature increase of a magnitude related or correlated or proportional to the SAR during each individual scan sequence. For example, during sequence 1 (S1), the SAR is low and there is a relatively small upward fluctuation in perceived temperature rise, while during for example sequence 2 (S2), the SAR is medium and there is a larger upward fluctuation in perceived temperature rise.

As illustrated in Fig. 3, there may be pauses or delay periods between consecutive scan sequences. During these delay periods, the patient will be still in the imaging zone of the scanner, but the next scan does not immediately start. This occurs because the technicians and radiologists typically review the acquired data in the previous scan sequence before starting the next sequence to determine if the data is of sufficient quality. During the pause periods, there is no active RF energy deposition in the body. Accordingly, the patient's skin temperature may start dropping. This in turn influences the thermal comfort of the patient. In particular, it will cause the patient to feel less warm or feel cold.

The drop in skin temperature between scan sequences is indicated by line 66 which shows predicted body temperature declining between scan sequences before rising again as the next scan sequence starts.

Fig. 3 further illustrates a computed ambient environment state sequence or program 72 executed throughout the duration of the scan, wherein sensory properties of the ambient environment state are controlled to be different during each scan sequence, adjusted in dependence upon the SAR during each scan sequence. There is one ambient environment state setting for each of the scan sequences. The ambient environment state settings are labelled AE1-AE8.

Each ambient environment state setting is determined by executing an instance of the thermoperceptual compensation operation. Each ambient environment state setting may be determined in advance before the scan begins, or may be computed in real time during the setting. Real time computation may allow for additional real-time data variables to be taken into account, as will be explained later. These may include for example measured physiological signals such as skin temperature, heart rate, respiration rate, sweating, skin conductance, vasoconstriction, vasodilation, and/or movement of the patient. Additionally, real time-time computation allows for real-time changes in the scan exam card to be taken into account. For example, scan sequences may be added or removed, and/or inter-scan delays/pauses can be adjusted to be shorter or longer.

The thermoperceptual compensation operation first comprises determining a direct or indirect indicator of an expected body (e.g. skin) temperature change between a first time point in the medical imaging scan and a later (second) time point in the medical imaging scan based on the scan information. In this case, for computing each environment state setting for each scan sequence, the second time point may be a time point at a start of the scan sequence, or during the scan sequence or at an end of the scan sequence. In this example, for simplicity, the SAR of the particular scan sequence is used as an indirect indicator of at least one component of the expected body temperature change.

With regards to body temperature change, a subject's body temperature at a given time may be taken to be correlated with a rate of energy absorption, i.e. correlated with the value of the SAR for a given scan sequence. This can be understood from the fact that the deposited RF energy causes local tissue warming, and thus a rate of energy deposition will be correlated with a body temperature change.

Thus, the SAR itself can simply be used as a proxy indicator for at least one component of an expected perceived temperature, based on the observation that rate of energy absorption correlates with change in body temperature. In this case for example, a change in SAR between the first time point and second time point may be used as the indicator of at least one additive component of an expected perceived temperature change. For example, expected SAR of an MR sequence can be used as a proxy for the body temperature change between a time before the scan has started (where SAR is zero) and a time period for which that scan sequence is being performed.

Thus, in a simplest case, for each scan sequence, an indirect indicator of the estimated body temperature change between a start time of the scan and a time, t, at or during that scan sequence may be estimated as:

$$\Delta T_p(t) \sim SAR(t)$$

where $\Delta T_p$ is the indirect indicator of estimated body temperature change at time $t$ and $SAR(t)$ is the SAR at time t.

In a more complex example, in addition to rate of energy deposition to the patient at any given time, also cumulative energy deposition to the patient might be taken into account. This factor is generally smaller than real time rate of energy deposition, because the homeostatic processes of the body act to dissipate excess heat from the body, such that there is typically only a relative small offset between rate of heat deposited and rate of heat expelled, such that there is only a relatively small cumulative build-up of heat energy in the body. Nonetheless, this gradual build-up of heat energy can be seen in the graph 66 of estimated perceived temperature rise in Fig. 3, where local (relatively large amplitude) fluctuations are superposed atop an underlying gradual upward trend, where the gradual upward trend represents the cumulative energy absorption over time.

Thus, in some embodiments, for each scan sequence, an indirect indicator of estimated body temperature change between a start time of the scan and a time, t, at or during that scan sequence may be estimated as a sum of a term proportional to an aggregate or cumulative energy absorption between the start of the scan and a time at or during the relevant scan sequence and a term proportional to the SAR for the relevant scan sequence. The two terms might be differently weighted. To compute the cumulative energy absorption at a time window corresponding to each scan sequence, the SAR information for the whole scan (at least up to the relevant scan sequence) may be needed, so that an indicator of a cumulative Figure can be computed or estimated.

For instance, according to one example, for each scan sequence, an indirect indicator of the estimated body temperature change between a start time of the scan and a time, $t_n$, at or during that scan sequence might be estimated as:

$$\Delta T_p\left(t_n\right) \sim \alpha\left[\int_{t=0}^{t=t_n} SAR(t)\,dt\right] + \beta\left[SAR(t_n)\right]$$

where $\Delta T_p$ is the indirect indicator of estimated body temperature change at time $t_n$ and $SAR(t)$ is the SAR at time $t_n$. The weightings $\alpha$ and $\beta$ may be adjusted as desired in order to differently weight the significance of cumulative energy deposition versus real time rate of energy deposition (as reflected by SAR(t)). The weighting $\alpha$ may be smaller than the weighting $\beta$. It is noted that the cumulative energy deposited does not reflect the cumulative energy retained in the body of the patient, since most of the energy is expelled through homeostatic processes. Hence, the cumulative energy deposition is an indirect indicator of cumulative energy retention by the patient. A lower weighting of $\alpha$ compared to $\beta$ can be used to reflect this.

Returning to the example of Fig. 3, it has now been described how an indicator of estimated body temperature change at the time window corresponding to each scan sequence may be computed. The second step of the thermoperceptual compensation operation for each scan sequence is to determine a setting for the ambient environmental state during that scan sequence in dependence upon the expected body temperature change so as to at least partially counter or offset a perceived body temperature change by the patient, i.e. to reduce the perceived temperature change, to target a more uniform perceived temperature throughout the scan.

This determination of the ambient environmental state may make use of a pre-defined thermoperception model which defines estimated mappings between different possible ambient environmental state settings and a change in perceived temperature by a person exposed to the relevant ambient environmental state.

In a simplest case, this model may for example simply comprise a lookup table which relates different ranges of values of the direct or indirect indicator of perceived temperature change with different specific settings of the ambient environmental state. When referring to a setting of the ambient environmental state, what is meant is a setting for the one or more variable sensory parameters of the ambient environmental state. Examples of variable sensory parameters include: an intensity or brightness or illuminance of a light output of an illumination system of the sensory output system, and/or a color temperature of a light output of the illumination system. Another possible variable is timing of the sensory outputs, e.g. a timing of light outputs. Other sensory modalities are also possible, as will be explained further below.

In the present example of Fig. 3, for simplicity, the sensory output system 52 is assumed to comprise simply an illumination system for generating a light output in the ambient environment, and wherein the illumination system is controllable to vary a color temperature of the light output.

As discussed above, to induce an increase in perceived temperature by the patient (the patient feels warmer), the light color temperature may be decreased (warmer color tones). Likewise, to induce a decrease in perceived temperature by the patient, the light color temperature may be increased (cooler color tones).

For simplicity, in the example of Fig. 3, only color temperature is adjusted as a variable setting of the ambient environment state for each scan sequence. In particular, during low SAR sequences, the ambient environment state is set with illumination having a warm color temperature (e.g. approximately 1200 K); during medium SAR sequences, the ambient environment state is set with illumination having medium warmth color temperature (e.g. approximately 5500 K); and during high SAR sequences, the ambient environmental state is set with illumination having cool color temperature (e.g. approximately 11000 K).

Thus, in this example, the thermoperception model may define mappings between different ranges of SAR for a given scan sequence and corresponding appropriate settings for variable parameters of the ambient environmental state to achieve at least partial compensation for temperature change. The variable parameters of the ambient environmental state may then simply be set to the settings defined by the thermoperception model mappings, in accordance with the known SAR for the scan sequence.

More preferably, the thermoperception model may define mappings between different ranges of the indicator of estimated body temperature change 66 as computed for a certain time point in the scan, $\Delta T_p$, and corresponding appropriate settings for variable parameters of the ambient environmental state to achieve at least partial compensation for temperature change.

Purely by way of one illustration, applying to the example of Fig. 3, a simple thermoperception model may take the form of a lookup table, for example of the form as set out in Table 1 below:

*Table 1*

| $\Delta T_p$ | Light color temperature (K) |
|---|---|
| Low | 1200 |
| Medium | 5500 |
| high | 11000 |

Thus, in this simple example, the values of the indicator of expected body temperature change, $\Delta T_p$ are classified into a reduced set of discrete classifications, for example, low, medium and high, and wherein the thermoperception model defines mappings between each expected body temperature change classification and a corresponding appropriate setting for variable parameters of the ambient environmental state to achieve at least partial compensation for the temperature change. Ranges of values of $\Delta T_p$ corresponding to each classification are pre-defined.

However, a more fine-grained model could also be used, wherein a larger number of different ranges of $\Delta T_p$ are associated in the model with particular settings for the illuminance and color temperature.

With regards to the particular values for the color temperature settings corresponding to each range of values of $\Delta T_p$, reference can be made to the literature studies referred to earlier. The results of these studies, in terms of ranges of color temperature and corresponding effect on perceived temperature are summarized in Table 2 below:

*Table 2*

| Paper | 'cool' condition | 'medium' condition | 'warm' condition | Effect on |
|---|---|---|---|---|
| Tsushima et al., 2020 | -Color temp: 5500K (=pure white) -Illuminance: 300 lx | N/A | -Color temp: 3000K -Illuminance: 800 lx | Higher perceived temperature in 'warm' color room than in 'cold' color room. |
| Golasi et al., 2019 | -Color temp: 11,530K, -Illuminance: 500 Lx | -Color temp: 4000K -Illuminance: 500 Lx | -Color temp: 1172K -Illuminance: 500 Lx | Light color influenced thermal perception (subjects felt warmer in warm vs. cooler lighting conditions) |
| Huebner et al., 2016 | -Color temp: 6500K -Illuminance: 495 Lx | N/A | -Color temp: 2700K -Illuminance: 550 Lx | Subjects put on more clothing in 'cold' vs. 'warm' light. |
| Bellia et al., 2021 | -Color temp: 6000K -Illuminance: 300 lx | N/A | -Color temp: 3000K -Illuminance: 300 lx | Light color influenced thermal perception (subjects felt warmer in warm vs. cooler lighting conditions) |
| Alfano et al., 2019 | -Color temp: 6000 K -Illuminance: 300 lx | | -Color temp: 3000 K -Illuminance: 300 lx | Light color influenced thermal sensation (subjects felt warmer in warm vs. cooler lighting conditions) |

Based on these literature results, it is proposed by the inventors that suitable ranges of values for the light color temperature corresponding to different classifications of expected body temperature change might be as follows:

*Table 3*

| $\Delta T_p$ | Light color temperature (K) |
|---|---|
| Low | 1200 - 3000 |
| Medium | 4000 - 5500 |
| High | 5500 - 11500 |

With regards to the ranges of values for the indicator of expected body temperature change, $\Delta T_p$, corresponding to the different classifications, these may be chosen as desired. By way of one example, where scan sequence SAR is used as the indirect indicator of body temperature change, $\Delta T_p$, then suitable ranges for $\Delta T_p$ for the different scan sequences may be for instance: High SAR: 2-4 W/kg; Medium SAR: 0.5-2 W/kg; Low SAR: less than 0.5 W/kg.

Thus, in this particular example, the mappings defined by the thermoperception model might be summarized as follows:

*Table 4*

| $\Delta T_p(t) \equiv$ SAR(t) (W/kg) | Light color temperature (K) |
|---|---|
| < 0.5 | 1200 - 3000 |
| 0.5-2.0 | 4000 - 5500 |
| 2.0-4.0 | 5500 - 11500 |

Thus, in this example, the values of the indicator of expected body temperature change, $\Delta T_p$ are classified into a reduced set of discrete classifications, for example, low, medium and high, for example corresponding to different ranges of values for $\Delta T_p$ and wherein the thermoperception model defines mappings between each expected body temperature change classification and a corresponding appropriate setting for variable parameters of the ambient environmental state to achieve at least partial compensation for the temperature change. More particularly, in this example, the thermoperception model defines mappings between the SAR of a given scan sequence and color temperature of light to output during the scan sequence, and wherein the mappings are such that color temperature of light to be applied increases as a function of increasing SAR.

Fig. 3 illustrates with line 68 a schematic representation of the perceived temperature of the patient as a function of time throughout the scan when the compensatory ambient environment states are activated during the scan. Line 68 therefore represents a corrected or compensated temperature perception of the patient.

It will be noted that the perceived temperature 68 of the patient still rises to some extent throughout the scan, but the total perceived temperature change across the whole scan is significantly lower than the expected (true) body temperature change 66, and the perceived temperature change is less locally variable that the true body temperature change. These effects result in improved thermal comfort and thermal satisfaction of the patient.

Although in the above described example of Fig. 3, there is one ambient experience (AE) state setting per scan sequence, in further examples, the AE state setting may be adjusted to change at different times during a single scan sequence. For example, there may be one AE setting for a first half and a second AE setting for a second half, or three different settings, each lasting a third of the scan sequence time duration. Additionally or alternatively, some AE settings may span more than one scan sequence in some embodiments.

Furthermore, in the example of Fig. 3, there is one AE setting which is maintained for the duration of the whole of each scan sequence and the delay period following the scan sequence. However, in further examples, there may be separate AE settings for the inter-scan delay periods. For example, the system may be configured to modify the light color temperature to a warmer color temperature after a pre-defined threshold delay period (without RF emissions being generated) has elapsed. This therefore helps to avoid the patient getting cold between scan sequences.

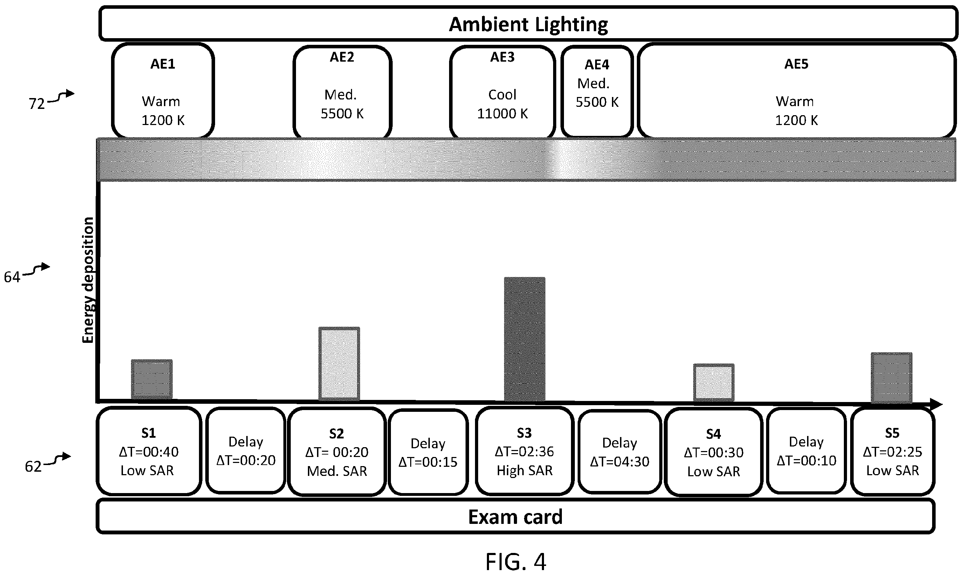

Fig. 4 shows a further example of a computed ambient environment state sequence or program 72 executed throughout the duration of the scan, wherein sensory properties of the ambient environment state are controlled to be different during each scan sequence, adjusted in dependence upon the SAR during each scan sequence. There is one respective ambient environment state setting (AE1, AE2, AE3) for each of the first three scan sequences, a further ambient environment state setting (AE4) which spans a delay period between the third and fourth ambient environment state settings and a final ambient environment state setting (AE5) which spans the final two scan sequences and a delay period separating the final two scan sequences.

The series of scan sequences forming the scan are indicated by arrow 62 in Fig. 4 and are labelled S 1-S5. Each scan sequence is labelled in accordance with its SAR, either low SAR, medium SAR or high SAR. S1 is low SAR, S2 is medium SAR, S3 is High SAR, S4 is low SAR, S5 is low SAR. By way of example, a high SAR might correspond to an SAR of between 2-4 W/kg, a medium SAR might correspond to an SAR of between 0.5-2 W/kg and a low SAR might correspond to an SAR of less than 0.5 W/kg. The time duration, $\Delta T$, of each sequence is further indicated for each scan sequence. Between each consecutive pair of scan sequences is a respective delay period. The time durations, $\Delta T$, of each delay period are indicated.

Above the outline of the scan sequences in Fig. 4 is a schematic bar graph 64 illustrating rate of energy deposition to the patient during each of the scans, i.e. the SAR. The height of each bar represents the rate of energy deposition to the patient during the particular one of the scan sequences 62 with which the bar is aligned.

It can be seen that during the low SAR scan sequences (S 1, S4, S5), a light output of an illumination system comprised by the sensory output system is set to a warm color temperature of 1200K. During the medium SAR sequences, the color temperature of the light output is set to a medium warm color temperature of 5500K. During the high SAR sequences, the light output of the illumination system is set to a cool color temperature of 1 1000K.

The delay periods between S1-S2 and S2-S3 are fairly short, at 20 seconds and 15 seconds respectively. By contrast the delay period between S3 and S4 is much longer, at 4 minutes and 30 seconds. During such a long delay period, following a high SAR sequence (which is warming the patient), the expected body temperature of the patient drops and the patient may begin to feel cold. For this reason, during the delay period between S3 and S4, an AE setting is applied in which medium warm light, of 5500K, is output. Sequences S4 and S5 both involve a low SAR, and the delay period between them is short, at 10 seconds. Thus, for this pair of sequences, a single continuous ambient environment setting of warm light (1200K) is applied spanning both S4 and S5 and the delay period in-between.

With regards to the illumination system of the sensory output system 52, this may comprise one or more light output devices which may be arranged in the room containing the imaging scanner, and/or arranged on the scanner itself. For example, there may be provided wall or ceiling mounted lighting to provide a light output in the ambient space of the room, this also being at least partially visible for example inside a bore of an MRI scanner. Additionally or alternatively, there may be one or more light sources provided on or around the scanner, arranged to provide a light output directed within the bore, so as to be visible to a patient inside the bore. The illumination system may further comprise a display device for presenting a 2D visual output, e.g. a display panel or projector. For example a display panel may be located within the bore of a scanner, visible to the patient when a scan is ongoing. This may present a visual output, e.g. a 2D or 3D visual output. This visual output may have a color content which is controllable and optionally a brightness which is controllable. For example, the visual output may comprise visual elements having a perceptible color temperature, wherein the color temperature is adjustable. A single uniform block of color might be presented or instead a color pattern may be presented. Additionally or alternatively, one or more images might be presented, where the images have color content.

In more advanced implementations, one or more other sensory aspects of the ambient environment state can be adapted, and/or other properties or statistics calculated from the sequence SARs can be used. For example, standard deviation, minimum, maximum, range, and difference between consecutive sequences can be computed, and these can be used to drive the ambient experience adaptations.

In other words, in some embodiments, the ambient environment state during each scan sequence may be adjusted not only based on using the thermoperception model but also based on one or more further rules or conditions. For example, in some embodiments, the thermoperceptual compensation operation may be configured such that, during the scan sequence having the highest SAR, the AE is configured so as to have the coolest light color compared to the AE of every other scan sequence of the imaging scan. In some embodiments, the AE settings of the set of scan sequences of the whole scan may be configured such that, before adjusting the AE to a cool light color setting, a warmer setting is applied to increase the effect of the cool settings. In some embodiments, the AE settings of the set of scan sequences of the whole scan may be configured such that the difference in color temperature between two consecutive scan sequences is proportional to the difference in the SAR of the two scan sequences.

In some embodiments, the estimate of body temperature change can be computed based on additional factors, such as level of bore ventilation provided, type of coil used duration of examination, patient clothing, and/or ambient temperature of the room.

Further optional features relating to the feature of determining the estimated body temperature change, and compatible with any embodiment of the invention, will now be described.

In accordance with some embodiments, to improve accuracy of estimated body temperature change, actual temperature measurements of the patient may be acquired during the scan. Temperature sensors that are non-metallic and non-magnetic can be used to measure the actual change in patient temperature. Some examples are fiber-optic temperature sensors, infrared thermometers, non-invasive sensors (thermistors or thermocouples placed on the skin or in the ear), and temperature sensitive stickers (placed on the skin and which change color as a function of patient temperature changes).

Thus, in some embodiments, the system may further comprise one or more temperature sensors for sensing a body temperature of the patient during the scan. The controller may be adapted to determine the indicator of an expected body temperature change based on at least a combination of the scan information (e.g. SAR, for example during each scan sequence) and a measure of patient body temperature at the second time point.

For example, in some embodiments, an ambient environment (AE) state setting for each scan sequence may be determined in advance based on the determined expected body temperature change of the patient between a start of the scan and a time point corresponding to the relevant scan sequence. Then, during execution of the scan, real body temperature measurements may be acquired using a skin temperature sensor, and the AE state setting may be adjusted in dependence upon any disparity between the expected body temperature change and the actual body temperature. For example, in the case that a thermoperception model is used which provides a mapping between body temperature change and AE state settings, then the real body temperature setting may be acquired and used to lookup an appropriate AE state setting corresponding to the body temperature change and adjust the AE state setting accordingly. In some embodiments, both the expected body temperature change and the actual body temperature change may be taken into account in determining an AE state setting, optionally with each given a different weighting, e.g. the expected body temperature change given a lower weighting than the actual body temperature change.

In some embodiments, the body temperature measurements may additionally or alternatively be used to control cooling equipment in the scan room (e.g. control a level of ventilation). In some embodiments, the body temperature measurements may additionally or alternatively be used to modify a sequence order of the scan sequences of the scan, and/or to control a pausing and/or termination of the scan. For example, if a body temperature increase rate is larger than expected by a threshold amount, the system may be configured to pause or terminate the scan.

In some embodiments, the thermoperception model may comprise a machine learning model trained to receive as input a target change in perceived temperature of a patient and generate as output settings for one or more sensory parameters of the sensory outputs of the sensory output system, e.g. change in color temperature, predicted to result in said change in perceived temperature.

The machine learning model may for example be an artificial neural network, e.g. a convolutional neural network.

In other examples, the machine learning model may be a trained regression model.

Other types of machine learning algorithm or model may also be considered.

With regards to training the model, the training data may comprise data compiled from historical MRI scanning sessions with one or more different patients during which the AE state is changed in defined ways and the patient is surveyed after each change to evaluate a change in the patient's perceived temperature. More concretely, the training data may comprise training input data values indicative of a patient's indicated change in their perceived body temperature and training output data values (ground truth values) indicative of a quantitative change in color temperature. In training, the model is trained to infer a predicted change in color temperature (output data values) which will result in an input target change in patient perceived temperature (input data values) .

In some embodiments, the model may be trained to receive a multiple-parameter input, wherein the multiple parameters include change in perceived temperature in addition to one or more further properties of the patient or the scanning room or the ambient environment state, such as physiological measurements (heart rate, respiration rate, sweating, skin conductance, vasodilation, vasoconstriction), an ambient temperature of the room, and/or data representing the SAR of a current scan sequence and cumulative scan duration.

To communicate the measured body temperature information in real-time, a wireless communication means may be provided for wireless communication of sensor data from the temperature sensor(s) to the controller. Alternatively, a wired connection is also possible.

In addition to or instead of measuring changes in patient body temperature, also measurements of ambient temperature in the environment of the medical imaging scanner may in some embodiments be acquired and used in computing the indicator of an expected body temperature change. In other words, the system may further comprise one or more ambient temperature sensors for sensing a temperature of the ambient environment, and the controller may be adapted to determine the indicator of an expected body temperature change based on at least a combination of the scan information and a measure of ambient environment temperature at the second time point.

For example, in the case of MRI imaging, MR compatible temperature sensors may be employed such as an infrared thermometer. In some examples, the infrared thermometer may be moveable, and wherein by moving and pointing the infrared thermometer to different objects or areas, the corresponding temperature can be measured.

In some embodiments, in addition to or instead of measuring temperature in the room of the imaging scanner, the controller may be adapted to retrieve real-time local weather information and use this information in computing the estimated perceived temperature changes.

In some embodiments, additional physiological signals can be used in estimating thermal discomfort, such as heart rate, blood flow to the skin, breathing rate, or ECG. In other words, the system may further comprise one or more physiological parameter sensors for sensing one or more physiological parameters of the patient, and wherein the controller is adapted to determine the indicator of an expected body temperature change based on at least a combination of the scan information and measurements of the one or more physiological parameters at the second time point.

Additionally or alternatively, in some embodiments, user input may be used in determining a perceived temperature change. The user might indicate via a hand control or via eye movements (using gaze tracking technology) an indication of their thermal discomfort.

The above descriptions have presented a relatively simple approach to determining estimated perceived temperature change of the patient, in particular using SAR and/or cumulative energy deposition as a proxy indicator for perceived temperature change. However, more sophisticated methods can also be used.

In particular, according to one set of embodiments, a thermophysiological model may be defined in advance which allows for simulating different perceived temperature changes in dependence upon a multiplicity of independent variables.

Reference is made in particular to the paper: van den Brink, Johan S. Thermal Effects Associated with RF Exposures in Diagnostic MRI: Overview of Existing and Emerging Concepts of Protection (hindawi.com). This paper details quantitative methods for estimating body temperature changes induced as a function of SAR and exposure duration. See in particular section 4 of the paper, and in particular Fig. 3 of the paper.

A further example thermophysiological model may additionally take other factors into account, such as ambient temperature, clothing, and well as the individual's thermoregulatory capabilities.

In some embodiments, one or more biological parameters of the patient may be taken into account in the modelling or simulation. such as any one or more of: age, gender, body mass, height, weight, body composition, metabolic base rate, resting heart rate, average activity level, and/or sleep patterns. This information for example can be obtained from a patient medical record system and/or from a medical scanner console.

In addition to these parameters, the modelling or simulation may also receive as inputs the scan information, such as information on the imaging protocol, e.g. scan sequence parameters, SAR, and/or procedure length. Optionally, equipment details and environment data may also be incorporated as inputs. With regards to environment data, this may include an indication of the season, the outside temperature, and/or the inside temperature. With regards to equipment details, this may include details regarding the available variable sensory parameters of the sensory output system, for example the types of sensory output device available and the variable parameters of each, e.g. brightness and color temperature of an illumination system.

One suitable example of modeling human temperature response to varying conditions, and capable of taking multiple factors into account is the Stolwijk approach. Here, inputs such as whole-body SAR, skin blood flow impairment, ambient temperature, relative humidity and insulation (clothing) information are used as inputs. The original Stolwijk model is described in the paper: Stolwijk JAJ. A mathematical model of physiological temperature regulation in man. Washington D.C.:; 1971. ReportNo.: NASA CR-1855.

Various developments of the model have been proposed. For example, one development is detailed in the following paper: Roelofsen, C. & Vink, Peter. (2016). Improvement of the Stolwijk model with regard to clothing, thermal sensation and skin temperature. Work. 54. 1009-1024.

Reference is also made to the following paper: Eleanor R. Adair, Larry G. Berglund, On the thermoregulatory consequences of NMR imaging, Magnetic Resonance Imaging, Volume 4, Issue 4, 1986, Pages 321-333. This describes a thermoregulatory model which can predict physiological heat loss responses in real time during an MRI scan as a function of selected ambient temperature (Ta), air movement (v), and rate of whole-body radiofrequency (RF) energy deposition (SAR).

The various additional parameters mentioned above can be used to create a more precise, fine-grained estimation of thermal discomfort than can be achieved SAR alone.

As discussed above, some embodiments of the invention utilize a thermoperception model to determine a change in ambient environment state which is to be performed responsive to a certain predicted change in body temperature during the scan. The thermoperception model may be configured to output an estimate of a change of ambient environment state which will result in a change in perceived temperature of the user which will compensate for a given expected change in actual body temperature, for example actual skin temperature.

Such a thermoperception model could be implemented using a simple lookup table, wherein the lookup table permits mapping from a value indicative of predicted change in body temperature to a particular set of settings for the ambient environmental state for compensating for the change, for example including at least a setting for a color temperature of an illumination output.

Such a thermoperception model may instead be implemented using a trained artificial neural network. The artificial neural network may be trained using training data which comprises patient temperature perception reports in combination with ambient environment state settings concurrent with said temperature perception reports.

In summary, according to one set of embodiments, the medical imaging scan may be an MRI scan, and wherein the scan information includes an expected Specific Absorption Rate (SAR) at one or more time points during the scan; and wherein the controller is adapted to determine the indicator of expected body temperature change based on use of a pre-defined thermophysiological model adapted to estimate RF-induced body temperature increases as a function of SAR and exposure duration. Additional factors, such as ambient temperature, clothing, and well as the individual's thermoregulatory capabilities can also be incorporated in the model.

In some embodiments, the controller may be further adapted to receive patient-specific temperature sensitivity information and wherein the determining the indicator of expected perceived temperature change is performed in part based on the patient-specific temperature sensitivity information.

For example, before the scan, the patient may provide input that can be used to further adjust the expected thermal discomfort. The collected patient information can include aspects such as sensitivity to temperature changes, as well as affect, anxiety, and other psychological, physical or demographic characteristics that are known to affect temperature and/or thermal perception.

In addition to this, patient input can be collected during the scan, using patient-interaction mechanisms such as audio input, audio output, a handheld control or eye tracking.

Based on the patient input, the patient temperature adjustment preferences may be determined and the ambient experience changed accordingly. This can be done either automatically or manually, e.g., by the MR technologist (who may receive a prompt asking them to make adjustments).

Various further optional details relating to the sensory output system, and compatible with any embodiment of the invention, will now be described.

As mentioned above, the sensory output system may comprise an illumination system comprising one or more light sources for generating a light output in the ambient environment. Ambient lighting can be adapted for example by changing the color of the lighting (or color temperature; K), such that cooler lights make people feel less hot.

Additionally or alternatively, the sensory output system may comprise a display device for presenting a 2D or 3D visual output. This may for example take the form of a display panel or projector. For example, a display panel can be provided inside the bore of a scanner. The 2D or 3D visual output can comprise visual elements which may be adjusted to adjust their color or content.

With regards to adjustment of the color, the 2D or 3D visual output can comprise visual elements having a perceptible color temperature, wherein the color temperature is adjustable. For example, the 2D or 3D visual output may comprise a presentation of visual imagery having perceptible color content, and wherein adjusting the color temperature of the output comprises adjusting the visual imagery.

With regards to adjustment of the content, the 2D or 3D visual output may comprise a presentation of visual imagery having a conceptual association with a certain range of thermal temperatures.

For example, imagery might be shown that is associated with coldness (e.g., snowy landscapes, cool water running down a stream) or warmth (e.g., a tropical beach, a fireplace with crackling fire).

Audio can be adapted to fit the image and light. For example, if warm elements are displayed, audio associated with these elements can be generated (e.g., crackling fire, running cold water, opening a fresh drink, etc.). In this case, the sensory output devices may include an audio output device.

To assist the patient transition from one state (e.g., feeling hot) to another (e.g., feeling less hot), it is also possible to show scenes that portray successful resolution of an aversive thermoceptive state. As an example, when a patient feels cold, the in-bore display screen of the display device might be controlled to show a scene in which a person (or anthropomorphic Figure such as a cartoon character) is shivering and looking cold before putting on a sweater, sitting by the fire and drinking hot chocolate. As a further example, if a person is feeling hot, the in-bore screen could show a scene of a person sweating and looking hot before diving in cold water on a sunny day, coming out of the water and drinking an ice-cold beverage.

In other words, the display device may be controlled to display visual representations of transitions between contrasting temperature scenes.

In addition to visually perceptible outputs, the sensory output system may be configured for generating sensory outputs of other sensory modalities.

One example is the use of smell, e.g., by exposing a patient to a smell associated with warmth-e.g., the smell of coconut, hot chocolate, or a campfire. To this end, the sensory output devices of the sensory output system may include an olfactory output device.

A further example is the use of tactile stimuli. For example, tactile stimuli may be administered to a patient to distract them from their aversive thermoceptive state. Tactile sensations such as vibration or tapping can alleviate feelings of thermal discomfort. To this end, in some embodiments, the sensory output devices of the sensory output system may include a tactile stimulus generator.

As discussed above, in preferred embodiments, the ambient environment state is varied throughout the scan so as to provide optimal thermoperceptive compensation for perceived temperature changes at different points throughout the scan. This therefore involves transitions in the ambient environmental state at various points throughout the scan. Sudden transitions could be jarring for the patient and could undermine the effectiveness of the sensory outputs.

Thus, according to at least one set of embodiments of the invention, it is proposed to implement functionality for further optimizing the transitions between ambient environmental states so as to make the transitions smoother and/or more gradual.

For example, where the change in the ambient environment state comprises at least a change in color temperature of a light output generated by an illumination system, the controller may be adapted to control the sensory output system such that a transition from a first ambient environment state to a second ambient environment state comprises a smooth transition of the light output through a color space.

From a technical perspective, most state of the art luminaires operate and/or are controlled in RGB color space, where each color is represented by a vector of three color values: Red, Green and Blue. However RGB is not a perceptually uniform color space. Therefore transitions from color A(R1,G1,B 1) to B(R2, G2, B2) may seem abrupt to an observer.

Accordingly, in accordance with at least one set of embodiments, it is proposed to convert color point A and B in RGB space to equivalent colors in CIELAB color space, thereby being transformed into A(L1,a1,b1) and B(L2, a2,b2), i.e.

$$A\ (R1,\ G1,\ B1) \rightarrow A(L1,\ a1,b1)$$

$$B\ (R2,\ G2,\ B2) \rightarrow B(L2,\ a2,b2)$$

It is proposed to further determine a continuous line in CIELAB space between points A and B, whereby points on that line will define transition color values in CIELAB to implement during the transition from A to B.

A plurality of values lying along the identified line in CIELAB space are extracted and converted back to RGB color space.

When moving between color A to color B in RGB color space, the illumination system is controlled to transition between color A and color B by controlling the illumination system to sequence through the converted RGB points, these corresponding to points along the line in CIELAB space. This way, a perceptually smooth color transition can be achieved from any first color to any other second color.

Conversion from CIELAB to RGB color space is a known procedure, and conversion equations are well known.

It is noted that, for most patient groups, thermal discomfort is aversive to the patient, and at least one aim of systems described herein is to relieve such discomfort. However, for specific patient groups (e.g., pregnant women or patients using medication that can affect thermoregulatory capacity), the risk of overheating in the scanner is larger than for most patients. As such, for these patient groups, it may be beneficial to control the ambient environmental state so as to increase perceived temperature during high-SAR sequences. Put differently: for sensitive patient groups (e.g., patients with thermoregulatory deficiencies), it may be beneficial not to use ambient environmental states which have the effect of making patients feel colder; instead, ambient environmental states may be used that signal high temperature to staff and patients. This may help patients and staff to be more aware of the potential risk of overheating.

Embodiments of the invention described above employ a controller comprising one or more processors. The controller may in general comprise a single processor or a plurality of processors. It may be located in a single containing device, structure or unit, or it may be distributed between a plurality of different devices, structures or units. Reference therefore to the controller being adapted or configured to perform a particular step or task may correspond to that step or task being performed by any one or more of a plurality of processing components, either alone or in combination. The skilled person will understand how such a distributed processing device can be implemented. The controller may include a communication module or input/output for receiving data and outputting data to further components.

The one or more processors of the controller can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. An environmental control system for use with a medical imaging scanner, the medical imaging scanner comprising an imaging apparatus adapted to acquire imaging data of an object located in an imaging zone, the system comprising:

a sensory output system comprising one or more sensory output devices adapted to generate a set of controllable sensory outputs, the combination of sensory outputs defining an ambient environment state sensorily perceptible by a patient located in the imaging zone, wherein the sensory output system is operable to vary the ambient environment state by controlling one or more variable sensory parameters of the sensory outputs, and wherein the combination of sensory outputs includes at least one or more visually perceptible outputs; and
a controller adapted to:

retrieve a thermoperception model defining a relationship between changes in the one or more variable sensory parameters of the sensory outputs and a resultant predicted change in perceived temperature by a person exposed to the ambient environment state comprising said sensory outputs; and
receive scan information indicative of medical imaging scan parameters as a function of time during a medical imaging scan for a patient, wherein the scan information includes an indicator of energy deposition to the patient by the scan as a function of time; and
perform a thermoperceptual compensation operation comprising:

determining a direct or indirect indicator of an expected body temperature change of the patient between a first time point in the medical imaging scan and a second later time point in the medical imaging scan based on the scan information; and
adjusting the ambient environment state at the second time point in dependence upon the expected body temperature change, based on using the thermoperception model to estimate a change in the ambient environment state setting which will at least partially counter a perception of the temperature change by the patient.

2. The system of claim 1, wherein the scan is composed of a set of individual scan sequences, each scan sequence associated with a different set of scan parameters,

wherein the first time point is a start time of the scan, and wherein the second time point is a start time of a particular scan sequence of the scan; or
wherein the first time point is a time coinciding with one scan sequence, and wherein the second time point is a time point coinciding with a later scan sequence.

3. The system of claim 2, wherein the controller is adapted to perform the thermoperceptual compensation operation for each scan sequence of the scan based on estimating an expected body temperature change induced by each scan sequence.

4. The system of any preceding claim, wherein the controller is adapted to perform the thermoperceptual compensation operation at a plurality of second time points across the duration of the scan, for example at regular time intervals throughout the scan.

5. The system of any preceding claim, wherein the sensory output devices include an illumination system for generating a light output in the ambient environment, and wherein the illumination system is controllable to vary a color temperature of the light output and/or to vary an illuminance or intensity of the light output.

6. The system of claim 5, wherein the thermoperception model is such that

light color temperature (K) is decreased and/or light illuminance (lux) is increased in order to increase perceived temperature by the patient; and
light color temperature (K) is increased and/or light illuminance (lux) is decreased in order to decrease perceived temperature by the patient.

7. The system of any preceding claim,

wherein the scan is an MRI scan, and wherein the scan information includes an expected Specific Absorption Rate (SAR) at one or more time points during the scan, and
optionally wherein the scan is composed of a set of individual scan sequences and the scan information includes an expected SAR for each scan sequence of the scan.

8. The system of claim 5, wherein a change in SAR between the first time point and second time point is used as at least one component of the indicator of an expected body temperature change.

9. The system of claim 8,

wherein the sensory output devices include an illumination system for generating a light output in the ambient environment; and
wherein the thermoperception model defines mappings between the SAR of a given scan sequence of the scan and a color temperature of light to output during the scan sequence, and wherein the mappings are such that color temperature of light to output increases as a function of increasing SAR.

10. The system of claim 8 or 9,

wherein the sensory output devices include an illumination system for generating a light output in the ambient environment;
wherein the first time point is a time point coinciding with one scan sequence, and wherein the second time point is a time point coinciding with a later scan sequence; and
wherein the thermoperception model is such that the adjusting the ambient environment state at the second time point in dependence upon the expected body temperature change comprises changing a color temperature of a light output by an amount which is a function of the change in SAR between the one scan sequence and the later scan sequence.

11. The system of any preceding claim,

wherein the sensory output system comprises a display device for presenting a visual output, e.g. a display panel or projector, and wherein the visual output comprises visual elements having a perceptible color temperature, wherein the color temperature is adjustable; and
optionally wherein the visual output comprises a presentation of visual imagery having perceptible color content, and wherein adjusting the color temperature of the output comprises adjusting the visual imagery.

12. The system of any preceding claim,

wherein the change in the ambient environment state comprises at least a change in color temperature of a light output generated by an illumination system, and
wherein the controller is adapted to control the sensory output system such that a transition from a first ambient environment state to a second ambient environment state comprises a transition of the light output through a path in color space.

13. The system of claim 12,

wherein the transition in the ambient environment state comprises at least a transition in RGB color of an RGB light output of the illumination system from a first color (A) in RGB space to a second color (B) in RGB space;
wherein performing the transition from color A to color B comprises:

converting color A and color B into points in the CIELAB color space
determining a continuous line through CIELAB color space between points A and B;
extracting a series of CIELAB color points lying along the CIELAB line and converting the extracted series of points to RGB color space, to thereby derive a series of RGB color points;
controlling the illumination system to transition between color A and color B by controlling the illumination system to sequence through the series of RGB color points.

14. An environmental control method for use during a medical imaging scan with a medical imaging scanner,

the medical imaging scanner comprising an imaging apparatus adapted to acquire imaging data of an object located in an imaging zone, and

the method comprising control of a sensory output system, the sensory output system comprising one or more sensory output devices adapted to generate a set of controllable sensory outputs, the combination of sensory outputs defining an ambient environment state sensorily perceptible by a patient located in the imaging zone, wherein the sensory output system is operable to vary the ambient environment state by controlling one or more variable sensory parameters of the sensory outputs, and wherein the combination of sensory outputs includes at least one or more visually perceptible outputs;

the method comprising:

receiving scan information indicative of medical imaging scan parameters as a function of time during a medical imaging scan for a patient, wherein the scan information includes an indicator of energy deposition to the patient by the scan as a function of time;

retrieving a thermoperception model defining a relationship between changes in the one or more variable sensory parameters of the sensory outputs and a resultant predicted change in perceived temperature by a person exposed to the ambient environment state comprising said sensory outputs; and

performing a thermoperceptual compensation operation comprising:

determining a direct or indirect indicator of an expected body temperature change of the patient between a first time point in the medical imaging scan and a later time point in the medical imaging scan based on the scan information; and

adjusting the ambient environment state at the second time point in dependence upon the expected body temperature change, based on using the thermoperception model to estimate a change in the ambient environment state setting which will at least partially counter a perception of the temperature change by the patient.

15. A computer program product comprising computer program code configured, when executed by a processor, to cause the processor to perform a method in accordance with claim 14.

10 ⟍

| Receive scan info | 12 |

↓

14 ⟍

| Expected body temp change | 16 |

↓

| Adjust AE state | 18 |

## FIG. 1

52

| Sensory output system |

32

34 — I/O

38 — Memory

36 — proc.

54

| Thermoperception model |

56

| Medical imaging scanner |

30

## FIG. 2

FIG. 3

FIG. 4

Europäisches
Patentamt

European
Patent Office

Office européen
des brevets

## EUROPEAN SEARCH REPORT

**Application Number**

EP 24 16 4713

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/055133 A1 (I GI TAE [KR] ET AL) 27 February 2014 (2014-02-27) * paragraphs [0007] - [0015], [0051] - [0061]; claims 1-35; figures 1-19 * ----- | 1-15 | INV. A61B5/00 A61B5/01 A61B5/055 A61F7/00 |
| A | US 7 702 375 B2 (SIEMENS AG [DE]) 20 April 2010 (2010-04-20) * column 1 - column 3; claims 1-21; figures 1-6 * ----- | 1-15 | G01R33/20 |

**TECHNICAL FIELDS SEARCHED (IPC)**

A61B
G01R
A61N
A61F

The present search report has been drawn up for all claims

| Place of search | Date of completion of the search | Examiner |
|---|---|---|
| The Hague | 12 August 2024 | Hirsch, Arthur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 4713

This annex lists the patent family members relating to the patent documents cited in  the above-mentioned European search report.
The members are as contained in the European Patent Office EDP file on
The European Patent Office is in no way liable for these particulars which are merely given for the purpose of information.

12-08-2024

| Patent document cited in search report | | Publication date | Patent family member(s) | | Publication date |
|---|---|---|---|---|---|
| US 2014055133 | A1 | 27-02-2014 | CN | 103622695 A | 12-03-2014 |
| | | | EP | 2700356 A2 | 26-02-2014 |
| | | | KR | 20140025711 A | 05-03-2014 |
| | | | US | 2014055133 A1 | 27-02-2014 |
| US 7702375 | B2 | 20-04-2010 | CN | 1550207 A | 01-12-2004 |
| | | | DE | 10322140 A1 | 16-12-2004 |
| | | | US | 2005004444 A1 | 06-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MYEONG SEONG KIM**. Investigation of Factors Affecting Body Temperature Changes During Routine Clinical Head Magnetic Resonance Imaging. *Iranian Journal of Radiology*
- **ADAIR, E.R.** ; **BERGLUND, L.G.** Predicted Thermophysiological Responses of Humans to MRI Fields. *Annals of the New York Academy of Sciences*, 1992, vol. 649, 188-200
- **SCHLADER et al.** Human temperature regulation when given the opportunity to behave. *Eur J Appl Physiol*, 2013, vol. 113, 1291-1301
- **HOFFMAN, H. G.** ; **RICHARDS, T. L.** ; **CODA, B.** ; **BILLS, A. R.** ; **BLOUGH, D.** ; **RICHARDS, A. L.** ; **SHARAR, S. R.** Modulation of thermal pain-related brain activity with virtual reality: evidence from fMRI. *Neuroreport*, 2004, vol. 15 (8), 1245-1248
- **MOGENSEN, M. F.** ; **ENGLISH, H. B.** The apparent warmth of colors. *The American Journal of Psychology*, 1926
- **TSUSHIMA, Y.** ; **OKADA, S.** ; **KAWAI, Y.** ; **SUMITA, A.** ; **ANDO, H.** ; **MIKI, M.** Effect of illumination on perceived temperature. *Plos one*, 2020, vol. 15 (8), e0236321
- **HARRY S. MAYES** ; **MARTINA NAVARRO** ; **LIAM P. SATCHELL** ; **MICHAEL J. TIPTON** ; **SOICHI ANDO** ; **JOSEPH T. COSTELLO**. The effects of manipulating the visual environment on thermal perception: A structured narrative review. *Journal of Thermal Biology*, 2023, vol. 112
- **KULVE et al.** The influence of light on thermal responses. *Acta Physiol.*, 2015, vol. 216 (2), 163-185
- **GOLASI et al.** Influence of lighting color temperature on indoor thermal perception: a strategy to save energy from the HVAC installations. *Energy Build.*, 2019, vol. 185, 112-122
- **STOLWIJK JAJ**. A mathematical model of physiological temperature regulation in man. *ReportNo.: NASA CR-1855*, 1971
- **ROELOFSEN, C.** ; **VINK, PETER.** Improvement of the Stolwijk model with regard to clothing, thermal sensation and skin temperature. *Work*, 2016, vol. 54, 1009-1024
- **ELEANOR R. ADAIR** ; **LARRY G. BERGLUND**. On the thermoregulatory consequences of NMR imaging. *Magnetic Resonance Imaging*, 1986, vol. 4 (4), 321-333